# EUROPEAN PATENT APPLICATION

(11) **EP 0 674 915 A1**
(43) Date of publication of application: **04.10.1995**
(21) Application number: 95400182.2
(22) Date of filing: 27.01.1995
(51) Int. Cl.: A61M 39/10

(54) **Lock fitting for transfusion equipment**

(30) Priority: 04.02.1994 IL 10855494
(71) Applicant: MEDIZIV MEDICAL PRODUCTS, Barkan Industrial Zone 44804 (IL)
(72) Inventor: Bar-Ziv, Samuel, Kfar Saba 44286 (IL)
(74) Representative: Bérogin, Francis

(57) **Abstract**

A conical lock fitting for fluid transfusion equipment comprising: a female socket member, a male plug member and a resilient annular gasket interposed between the male and the female members. The gasket is retained between a wider proximal portion of the lumen of the female member and a narrower distal portion of the lumen.

## Description

### FIELD OF THE INVENTION

The present invention concerns medical equipment for transfusion of fluids. More specifically, the invention provides an improved conical lock fitting for said medical equipment.

### BACKGROUND OF THE INVENTION

Standard conical lock fittings with a 6% (Luer) taper are conventionally used in medical transfusion equipment, such as syringes, needles, catheters, blood transfusion sets, dialysis etc. The requirements for such conical lock fittings are specified in the ISO International Standard 594 (1986).

Such lock fittings, comprising a male plug member and a female socket member, are most commonly and widely used, but suffer from the disadvantage that when the female and male members are coupled, a small empty space generally remains between these two members. The fluid filling this space when a transfusion is performed, may remain comparatively stagnant and become a potential source of trouble. For example, blood might clot in this space and the clots might enter the blood vessels of the body, or the clots might block the lumen and stop the flow of the transfusion fluids. Drugs and/or other substances, present in a transfusion liquid, might precipitate in this space and get carried into the body.

It is therefore the object of the present invention to provide an improved conical lock fitting which would overcome the above disadvantages of conventional conical lock fittings.

### SUMMARY OF THE INVENTION

The object of the present invention is achieved by a conical lock fitting for fluid transfusion equipment comprising a female socket member and a male plug member, characterized in that the lock fitting further comprises a resilient annular gasket interposed between said male and said female members and retained between a wider proximal portion of the lumen of said female member and a narrower distal portion of said lumen.

Said conical lock fitting may further comprise a resilient annular gasket interposed between said male and said female members and retained by an annular shoulder formed between a wider proximal portion of the lumen of said female member and a narrower distal portion of said lumen.

When the male and female members are coupled, the tip of the conical portion of the male member bears against the inner resilient gasket, leaving no empty space between the male and female members in the interior of the lock fitting, thereby avoiding the above-mentioned risk of blood clotting or blocking by other solid precipitates.

The present invention also provides a female socket member for use in a conical lock fitting, wherein the proximal portion of the lumen is larger in diameter that the distal portion of said lumen so as to form an inner annular shoulder between said portions.

The present invention further provides a female socket member for use in a conical lock fitting having a resilient annular gasket inserted therein and retained by said shoulder.

The gasket may be made of any suitable resilient polymer which is chemically inert to the fluids to be passed through the system. Suitable materials are, e.g. silicone, soft P.V.C., natural or synthetic rubber, latex or polyurethane.

### BRIEF DESCRIPTION OF THE DRAWINGS

The present invention will be further described in detail, with reference to the non-limiting attached drawings, in which:

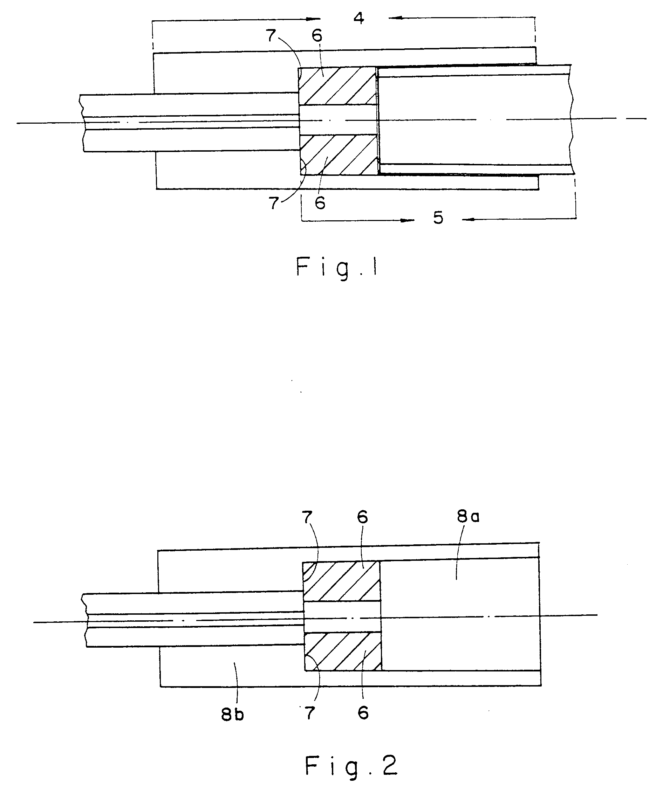

**Fig. 1** is a schematic partial view of the conical lock fitting according to the invention; and
**Fig. 2** is a schematic view of a female socket member having an annular gasket inserted therein.

### DETAILED DESCRIPTION OF THE INVENTION

The present invention provides an improved conical fitting for fluid transfusion equipment which comprises a male plug member **5**, a female socket member **4** and a resilient annular gasket **6** which is interposed between the male **5** and the female **4** members.

In the female socket member **4**, which is provided by the invention for use in the conical lock fitting, the proximal portion of the lumen **8a** is larger in diameter than the distal portion of the lumen **8b**, thereby, forming an inner annular shoulder **7** between the portions. The annular gasket **6**, when inserted in the female member **4**, is retained by the shoulder **7**.

Usually in conventional lock fittings, when the members are locked, an empty space remains between the male and the female members. This space is filled, according to the invention, by the gasket **6**, thereby avoiding the risk of blood clotting or blocking by other solid precipitates which are present in the transfusion fluids.

The gasket **6** may be made of any resilient polymer which size is only slightly changed by pressure, but the passage of fluids through the lumen of the lock fitting is still possible. Such polymers may be soft P.V.C., rubber, latex or polyurethane, preferably silicone.

## Claims

1. A conical lock fitting for fluid transfusion equipment comprising a female socket member **4** and a male plug member **5**, characterized in that the lock fitting further comprises a resilient annular gasket **6** interposed between said male and said female members **4**, **5** and retained between a wider proximal portion **8a** of the lumen of said female member **4** and a narrower distal portion **8b** of said lumen.

2. A conical lock fitting according to Claim 1 further characterized by an inner annular shoulder **7** formed between a wider proximal portion **8a** of the lumen of said female member **4** and a narrower distal portion **8b** of said lumen.

3. A conical lock fitting according to Claim 2, characterized in that the proximal portion **8a** of the lumen of said female socket member **4** is larger in diameter than the distal portion **8b** of said lumen so as to form an inner annular shoulder **7** between said portions **8a**, **8b**.

4. A conical lock fitting according to Claim 3, characterized in that said female socket member **4** comprises an annular gasket **6** inserted therein and is retained by said shoulder **7**.

5. A conical lock fitting according to Claim 4, characterized in that annular gasket **6** of said female socket member **4** is made of a resilient polymer.

6. A conical lock fitting according to Claim 5, characterized in that annular gasket **6** of said female socket member **4** is made of silicone, soft P.V.C., latex, rubber or poly-urethane.
